# FASCICULE DE BREVET EUROPEEN

(11) **EP 2 196 238 B1**
(45) Date de publication et mention de la délivrance du brevet: **25.05.2011**
(21) Numéro de dépôt: 09173444.2
(22) Date de dépôt: 19.10.2009
(51) Int. Cl.: A61N 1/362, A61N 1/365

(54) **Dispositif médical actif du type prothèse cardiaque implantable, comprenant des moyens de stimulation auriculaire antitachycardique et de stimulation ventriculaire antibradycardique**
Aktive implantierbare medizinische Herzvorrichtung mit antitachykarder Vorhofstimulierung und antibradykarder Ventrikelstimulierung
Active implantable medical cardiac device, including means of antitachycardiac auricular stimulation and antibradycardiac ventricular stimulation

(30) Priorité: 12.12.2008 FR 0806968
(43) Date de publication de la demande: 16.06.2010
(73) Titulaire: ELA MEDICAL, 92541 Montrouge (FR)
(72) Inventeur: Vincent, Elodie, 92160, Antony (FR)
(74) Mandataire: Dupuis-Latour, Dominique

(56) Documents cités:
- WO-A-95/03086
- WO-A-95/13741
- WO-A-95/27531
- WO-A-2008/054261
- US-A- 5 549 652

## Description

L'invention concerne les "dispositifs médicaux implantables actifs" tels que définis par la Directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Elle concerne plus particulièrement ceux de ces dispositifs qui possèdent un mode de thérapie "ATP" (Anti-Tachycardia Pacing), c'est-à-dire une stimulation programmée haute fréquence de l'oreillette ou mode analogue (stimulation 30 Hz notamment). Ce type de thérapie est indiqué pour traiter certaines arythmies provoquées par des perturbations des potentiels d'action au niveau de l'oreillette. La thérapie par stimulation de l'oreillette peut être appliquée en cas de détection d'un rythme cardiaque rapide anormal d'origine auriculaire (fibrillation auriculaire ou autre arythmie auriculaire). Elle sera appelée par la suite "stimulation auriculaire antitachycardique".

Le WO95/03086 A1 divulgue un tel dispositif permettant d'appliquer si nécessaire une stimulation ATP au porteur de l'implant.

De façon générale, la décision d'appliquer une thérapie antitachycardique, et le choix de cette thérapie (application d'un choc de défibrillation, ou bien stimulation antitachycardique de type ATP ou analogue) est prise par un algorithme de détection et de classification des différentes tachyarythmies en fonction de plusieurs critères de discrimination tels que la fréquence ventriculaire, la stabilité des intervalles ventriculaires (intervalles RR), l'analyse de l'association auriculo-ventriculaire (révélée par la stabilité de l'intervalle PR) ou encore le mode de démarrage des tachycardies (présence d'une accélération brusque et cavité d'origine, ventriculaire ou auriculaire).

Certaines de ces tachycardies peuvent être traitées en activant une stimulation antitachycardique (ATP) auriculaire.

Cependant, lors de la délivrance d'une stimulation antitachycardique au niveau de l'oreillette, le dispositif n'est plus capable de détecter les événements ventriculaires, du fait de la durée des périodes réfractaires ventriculaires post-stimulation auriculaire, et de l'intervalle entre deux stimulations auriculaires.

Or la contraction ventriculaire au cours de ces épisodes d'arythmie peut être très irrégulière, voire inexistante, entraînant l'apparition de pauses ventriculaires. Il est donc important de maintenir une détection de l'activité du ventricule pendant l'application de la stimulation auriculaire antitachycardique afin de pouvoir, si nécessaire, stimuler le ventricule si aucun événement spontané ventriculaire n'est détecté (on parlera par la suite de "stimulation ventriculaire antibradycardique" pour désigner cette stimulation conditionnelle du ventricule).

Plus précisément, des perturbations des potentiels d'action au niveau de l'oreillette et/ou du ventricule peuvent entraîner des arythmies ventriculaires. Celles-ci sont généralement classifiées en tant que tachycardies supraventriculaires (TSV) ou tachycardies ventriculaires (TV). Les TSV sont caractérisées par un rythme anormal au niveau de l'oreillette ou du noeud auriculo-ventriculaire. Les plus communes sont les *flutters* auriculaires et les fibrillations auriculaires :
- pendant les *flutters* auriculaires, les potentiels d'action ont un cheminement intra-auriculaire circulaire, ce qui entraîne une accélération de la fréquence de contraction de l'oreillette avec une conduction auriculo-ventriculaire intermittente (2:1 ou 3:1 par exemple) ;
- quant aux fibrillations auriculaires, ce sont des arythmies communes qui correspondent à des dépolarisations anarchiques des oreillettes, entraînant une activité parfois rapide et souvent irrégulière des ventricules réduisant de ce fait l'efficacité hémodynamique de la contraction de ces derniers.

Lors de ces arythmies auriculaires, le patient peut ressentir des palpitations, se plaindre de malaises, de dyspnée et de douleurs thoraciques. Il est donc important de les traiter dans certains cas, et l'application d'une stimulation auriculaire antitachycardique est l'une des solutions de traitement de ces arythmies.

Or en présence d'une stimulation de l'oreillette - et donc, notamment, pendant l'application d'une thérapie auriculaire antitachycardique - la détection électrique de l'activité ventriculaire par l'implant peut être masquée par les périodes réfractaires post-stimulation auriculaire.

Une première solution consiste à délivrer systématiquement une stimulation ventriculaire pendant la durée de cette thérapie de stimulation auriculaire antitachycardique. Cette stimulation ventriculaire sera toutefois une stimulation asynchrone, en raison des périodes réfractaires consécutives à la stimulation et des périodes de *blanking*, correspondant à la déconnexion des circuits de détection de l'amplificateur pour permettre la décharge de l'énergie accumulée à l'interface coeur/électrode après la stimulation). Du fait de cet asynchronisme, le risque existe de stimuler sur l'onde T, avec comme possible conséquence le déclenchement d'une arythmie ventriculaire.

L'un des buts de l'invention est de remédier à ces difficultés, en proposant une nouvelle technique de détection de l'activité ventriculaire lors de l'application d'une stimulation auriculaire antitachycardique, de manière à pouvoir si nécessaire appliquer au ventricule, pendant la durée de cette thérapie auriculaire; une stimulation antibradycardique qui soit à la fois contrôlée (appliquée seulement à bon escient, et non systématiquement) et synchrone, de manière à éviter tout risque de déclenchement d'une arythmie ventriculaire.

Essentiellement, l'invention propose de détecter la contraction, ou l'absence de contraction, du ventricule par recherche d'une activité mécanique de celui-ci par le biais d'un signal d'activité mécanique, notamment un signal d'accélération endocardiaque délivré par un capteur accélérométrique implanté, ou tout autre capteur permettant de mesurer l'activité mécanique des ventricules (pression, bioimpédance intracardiaque, etc.). Un tel capteur d'accélération endocardiaque pourra être présent sur la sonde ventriculaire, ou sur une autre sonde dotée d'un tel capteur, sonde placée soit directement dans le ventricule (sonde endocavitaire), soit en une autre position, par exemple dans l'oreillette, permettant de recueillir le signal d'accélération endocardiaque (signal EA) représentatif des contractions du ventricule.

En d'autres termes, l'invention propose essentiellement d'utiliser, pendant la stimulation antitachycardique de l'oreillette, un signal fonctionnel (le signal EA) représentatif de la contraction mécanique du ventricule, en lieu et place d'un signal issu de la propagation électrique de l'onde de dépolarisation. La détection de l'activité mécanique du ventricule viendra ainsi relayer la détection de l'activité électrique de celui-ci pendant la durée de la thérapie auriculaire antitachycardique.

L'invention propose à cet effet un dispositif médical actif implantable du type prothèse cardiaque de stimulation, resynchronisation et/ou défibrillation tel que divulgué par le WO95/03086 A1 précité, c'est-à-dire comportant : des moyens de détection auriculaire, aptes à détecter la survenue d'événements auriculaires spontanés ; des moyens de détection ventriculaire, aptes à détecter la survenue d'événements ventriculaires spontanés ; des moyens de stimulation antitachycardique, aptes à délivrer des séquences d'impulsions de stimulation de faible énergie à une fréquence supérieure au rythme sinusal du patient en cas d'arythmie ; des moyens de calcul d'un intervalle d'échappement ventriculaire ; et des moyens de stimulation ventriculaire antibradycardique, aptes à délivrer conditionnellement une impulsion de stimulation ventriculaire, en l'absence d'événement ventriculaire spontané détecté par les moyens de détection ventriculaire à l'issue de l'intervalle d'échappement ventriculaire. Le dispositif comprend également : un capteur d'activité mécanique du myocarde, apte à délivrer un signal d'activité mécanique représentatif des mouvements produits par les contractions cycliques de la cavité ventriculaire ; et des moyens de détection ventriculaire, comprenant : des moyens principaux de détection, aptes à détecter un potentiel électrique de dépolarisation ventriculaire spontanée (R), et des moyens auxiliaires de détection, aptes à reconnaître et isoler dans le signal d'activité mécanique délivré par le capteur une composante associée à une contraction ventriculaire.

De façon caractéristique de l'invention, les moyens de stimulation antitachycardique sont des moyens de stimulation auriculaire, et les moyens auxiliaires de détection sont mis en oeuvre en cas d'activation des moyens de stimulation auriculaire antitachycardique, de manière à relayer les moyens principaux de détection en présence de périodes réfractaires post-stimulation auriculaire occultant la détection de l'activité électrique du ventricule par les moyens principaux de détection. En outre, les moyens de calcul de l'intervalle d'échappement comprennent : des moyens pour calculer une première valeur d'intervalle d'échappement (IE_{RR}) à partir des instants successifs de survenue des potentiels de dépolarisation ventriculaire spontanée (R) détectés par les moyens principaux de détection ; des moyens pour calculer une seconde valeur d'intervalle d'échappement (IE_{PEA}) à partir des instants successifs de survenue desdites contractions ventriculaires détectées par les moyens auxiliaires de détection ; et des moyens pour sélectionner la seconde valeur d'intervalle d'échappement en cas d'activation des moyens de stimulation auriculaire antitachycardique.

Les moyens pour calculer ladite seconde valeur peuvent limiter la valeur d'intervalle d'échappement calculée à une valeur minimale prédéterminée, et par ailleurs moyenner une pluralité de valeurs d'échappement relevées au cours de cycles successifs.

Le capteur d'activité mécanique du myocarde est de préférence un capteur d'accélération apte à délivrer un signal d'accélération endocardiaque, les moyens auxiliaires de détection étant alors aptes à reconnaître et isoler dans le signal d'accélération endocardiaque une composante correspondant à un pic d'accélération endocardiaque associé à ladite contraction ventriculaire. Ce capteur d'accélération peut être un capteur endocavitaire, un capteur épicardique, ou bien un capteur de mouvement d'une paroi du myocarde.

En variante, le capteur d'activité mécanique du myocarde peut être un capteur de pression ou un capteur d'impédance intracardiaque.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre un exemple de signal d'accélération endocardiaque EA recueilli au cours de trois cycles cardiaques successifs.
La Figure 2 est une série de trois chronogrammes illustrant différents signaux caractérisant l'activité cardiaque au cours d'un cycle donné.
La Figure 3 illustre un exemple, pris sur la durée de cinq cycles cardiaques successifs, des signaux d'accélération endocardiaque et d'électrogramme, expliquant la manière dont l'invention gère la stimulation ventriculaire sur la base d'un signal d'accélération endocardiaque pendant la durée d'une stimulation auriculaire antitachycardique.
La Figure 4 est un ordinogramme expliquant les différentes étapes de la gestion de cette stimulation ventriculaire.

On va maintenant décrire un exemple de réalisation du dispositif de l'invention. En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply* et *Paradym* produits et commercialisés par ELA Médical, Montrouge, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail. On sait recueillir un signal d'activité mécanique, notamment un signal d'accélération endocardiaque comme cela est par exemple décrit dans le EP-A-0 515 319 (Sorin Biomedica Cardio SpA) qui décrit une sonde endocavitaire pourvue d'une électrode distale de stimulation implantée au fond du ventricule, intégrant en outre un micro-accéléromètre permettant de mesurer l'accélération endocardiaque.

Le signal d'accélération endocardiaque ainsi mesuré au cours d'un cycle cardiaque forme, entre autres, deux pics correspondant aux deux bruits majeurs qu'il est possible de reconnaître dans chaque cycle d'un coeur sain. Le EP-A-0 655 260 (Sorin Biomedica Cardio SpA) décrit une manière de traiter le signal d'accélération endocavitaire délivré par le capteur situé en bout de sonde pour en dériver, notamment, la position temporelle et l'amplitude ces deux valeurs de pic d'accélération endocardiaque, utiles notamment pour la détection de troubles cardiaques et le déclenchement ou non d'une thérapie de défibrillation.

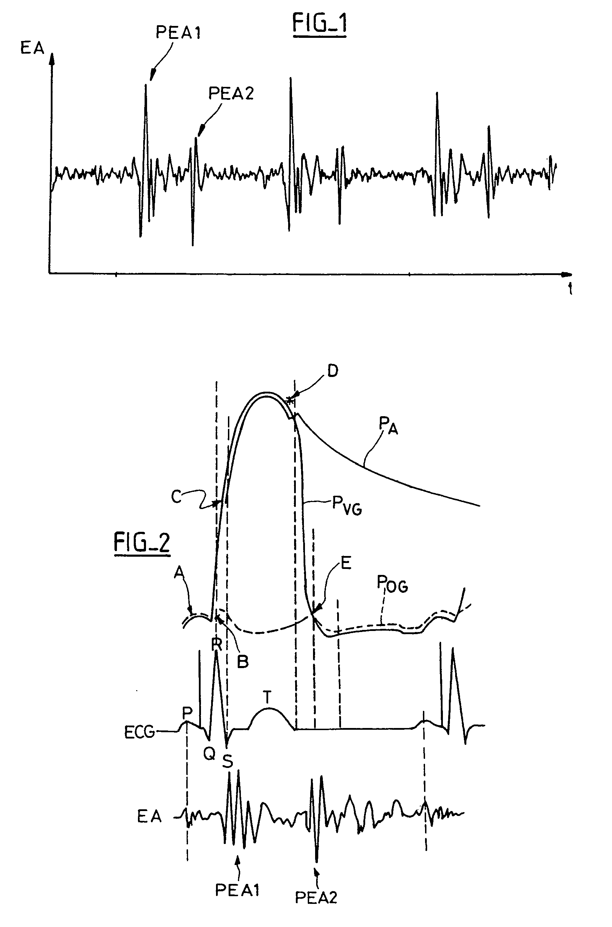

Sur la Figure 1, on a représenté les variations au cours de trois cycles cardiaques consécutifs de l'accélération endocardiaque (EA), mesurée par un capteur tel que celui décrit dans le EP-A-0 515 319 précité, intégré à une tête de sonde endocavitaire placée au fond du ventricule.

Comme on peut le voir, le signal EA forme au cours d'un cycle cardiaque deux pics principaux, correspondant aux deux bruits majeurs (sons S1 et S2 du phonocardiogramme) qu'il est possible de reconnaître dans chaque cycle d'un coeur sain. Plus précisément :
- le premier pic d'accélération endocardiaque ("PEA1") correspond à la fermeture des valvules mitrale et tricuspide, au début de la phase de contraction ventriculaire isovolumique (systole). Ses variations sont étroitement liées aux variations de la pression dans le ventricule (l'amplitude du pic PEA1 étant, plus précisément, corrélée au maximum positif de la variation de pression dP/dt dans le ventricule gauche) ;
- le second pic d'accélération endocardiaque ("PEA2"), quant à lui, correspond à la phase de relaxation ventriculaire isovolumique, et il est produit par la décélération brusque de la masse sanguine en mouvement dans l'aorte.

Le signal EA contient également deux autres composantes, d'amplitude très inférieure, appelées EA3 et EA4, correspondant aux sons S3 et S4 du phonocardiogramme.

La Figure 2 illustre les différents signaux caractérisant l'activité du coeur au cours d'un cycle cardiaque, avec : le profil des pressions intracardiaques, un relevé d'électrocardiogramme de surface (ECG), et les variations du signal d'accélération endocardiaque (EA).

Sur le profil des pressions intracardiaques, la caractéristique P_{A} illustre les variations de la pression aortique, P_{VG} celles du ventricule gauche et P_{OG} celles dans l'oreillette gauche. Les points A à E correspondent aux différentes phases suivantes : A, contraction de l'oreillette gauche ; B, fermeture de la valvule mitrale ; C, ouverture de la valvule aortique ; D, fermeture de la valvule aortique ; et E, ouverture de la valvule mitrale.

Le signal ECG comprend successivement : l'onde P correspondant à la dépolarisation de l'oreillette, le complexe QRS correspondant à la dépolarisation du ventricule et l'onde T de repolarisation ventriculaire.

Le signal d'accélération endocardiaque EA, quant à lui, peut se décomposer de la manière suivante : la contraction de l'oreillette (onde P) est suivie par la composante EA1, qui débute à la suite du complexe QRS et est engendrée par une combinaison de la fermeture des valves auriculo-ventriculaires, de l'ouverture des valves semi-lunaires et de la contraction du ventricule gauche. La composante EA2 qui lui fait suite, quant à elle, accompagne la fin de la systole ventriculaire et elle est générée par la fermeture des valves semi-lunaires.

La position temporelle des pics d'accélération endocardiaque, notamment du pic PEA1 de la composante EA1, peuvent être déterminées à chaque cycle par un traitement approprié du signal délivré par le capteur d'accélération.

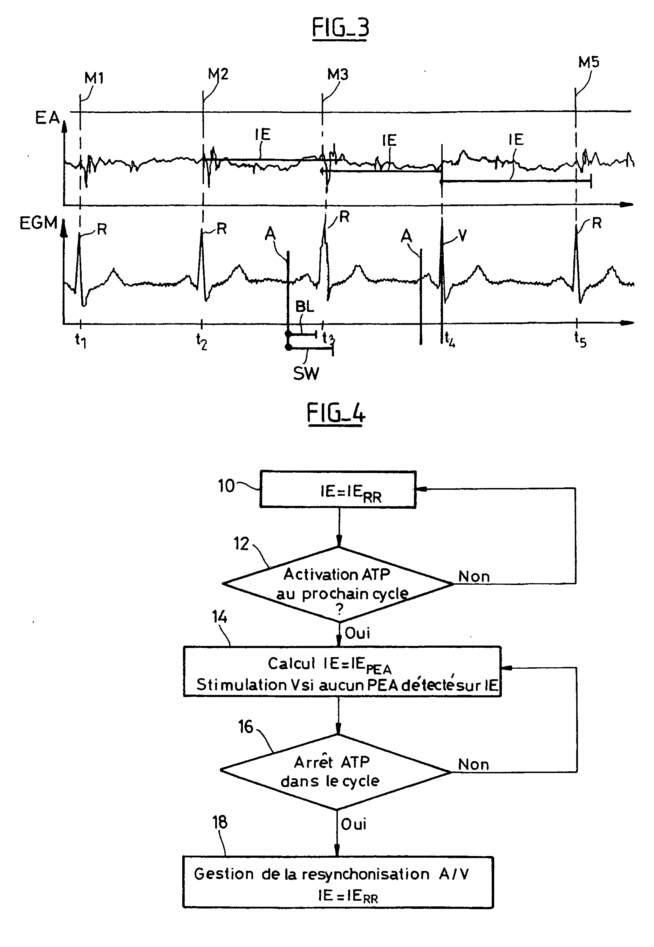

Sur la Figure 3, on a illustré sur la ligne du haut les marqueurs temporels successifs M₁, M₂, M₃ ... délivrés par l'appareil, correspondant aux instants de début des pics PEA1 ainsi détectés sur le signal EA par des moyens en eux-mêmes connus.

La seconde ligne donne le signal EA à partir duquel sont obtenus ces marqueurs, tandis que la troisième ligne est un tracé du signal EGM correspondant à l'électrogramme.

De façon générale, la stimulation antibradycardique du ventricule implique le calcul par le dispositif d'un "intervalle d'échappement" (IE), qui est l'intervalle de temps, compté après un événement ventriculaire (détection d'une dépolarisation spontanée R ou bien stimulation V), à l'issue duquel une stimulation est délivrée au ventricule si aucun événement spontané n'a été détecté dans cette même cavité.

Cet intervalle d'échappement est habituellement calculé à partir des instants successifs de survenue des potentiels de dépolarisation ventriculaire spontanée (pics R), sur la base des événements électriques détectés dans le ventricule, et déclenché sur chaque événement ventriculaire spontané ou stimulé. Cette valeur sera par la suite dénommée "intervalle d'échappement électrique" et noté IE_{RR}.

De façon caractéristique de l'invention, le dispositif calcule, concurremment à l'intervalle d'échappement électrique IE_{RR}, une deuxième valeur d'intervalle d'échappement, ci-après désignée "intervalle d'échappement mécanique" et notée IE_{PEA}, se basant non pas sur les intervalles RR mais sur les instants séparant les pics PEA successifs.

En effet, à partir de la détection cycle à cycle du PEA, il est possible de déterminer et surveiller l'intervalle PEA-PEA (c'est-à-dire l'intervalle séparant les marqueurs Mᵢ et Mᵢ₊₁). Ce suivi peut intervenir :
- en continu ; ou
- seulement en cas de suspicion d'une arythmie auriculaire soutenue (situation correspondant par exemple à un passage du dispositif en mode de repli) ; ou encore
- seulement à partir de la dernière détection ventriculaire (recueil d'une activité spontanée ayant son origine dans le ventricule, correspondant à un pic R sur l'électrogramme EGM) précédant le déclenchement d'une stimulation auriculaire antitachycardique.

L'intervalle d'échappement mécanique IE_{PEA} est déclenché sur chaque événement ventriculaire spontané ou stimulé, et calculé sur des règles similaires à celles utilisées pour le calcul de l'intervalle d'échappement électrique IE_{RR}, mais en se basant sur les intervalles PEA-PEA successifs au lieu des intervalles RR.

La Figure 4 illustre les différentes étapes d'un exemple d'algorithme de gestion de la stimulation ventriculaire mettant en oeuvre les enseignements de l'invention.

À l'origine (étape 10), la valeur de l'intervalle d'échappement utilisé par le dispositif est la valeur de l'intervalle d'échappement électrique IE_{RR}, déclenché sur détection d'un événement ventriculaire, spontané (R) ou stimulé (V).

Sur le dernier cycle ventriculaire précédant l'application d'une thérapie auriculaire antitachycardique (test 12), le dispositif démarre un intervalle d'échappement IE.

Mais l'intervalle d'échappement électrique IE_{RR} ne pourra plus être utilisé pendant la thérapie auriculaire, du fait de la stimulation auriculaire rapide, avec des périodes réfractaires ventriculaires susceptibles de masquer la détection électrique d'une dépolarisation spontanée du ventricule. Cette situation correspond au deuxième cycle cardiaque sur la Figure 3 : la stimulation auriculaire indiquée par le marqueur A déclenche à la fois une période BL de blanking post-stimulation auriculaire et une fenêtre de sécurité SW, périodes pendant lesquelles aucune détection ventriculaire ne sera détectée par l'appareil. En particulier, la dépolarisation spontanée R à l'instant t₃ ne pourra pas être détectée par l'amplificateur de détection des dépolarisations électriques du ventricule. En revanche, cet événement survenant à l'instant t₃ pourra être détecté par l'analyse du signal EA, en raison de la présence d'un pic PEA1, donnant un marqueur temporel M₃.

On peut donner à l'intervalle d'échappement IE (étape 14) :
- soit la dernière valeur de IE_{RR} (ou bien la moyenne des quatre dernières valeurs IE_{RR}), dans le cas où le PEA n'est pas monitoré en continu ni pendant l'arythmie auriculaire,
- soit la dernière valeur de IE_{PEA} (ou la moyenne des quatre dernières valeurs IE_{PEA}), si le PEA est déjà monitoré avant le début de la thérapie auriculaire, notamment lorsque ce PEA est monitoré de façon continue par le dispositif.

A la fin de l'intervalle d'échappement IE, si le dispositif n'a détecté aucun PEA, une stimulation ventriculaire est délivrée, et un nouvel intervalle d'échappement IE_{PEA} est calculé et démarré.

Cette situation correspond par exemple au quatrième cycle de l'exemple illustré sur la Figure 3 : alors que pour les cycles précédents une contraction ventriculaire avait été détectée (marqueurs M₁, M₂ et M₃), au cycle suivant aucune contraction n'est détectée (absence de marqueur M₄ révélant une contraction du ventricule). Une stimulation ventriculaire (marqueur V) est alors appliquée par le dispositif pour provoquer la contraction du ventricule qui n'a pu se contracter spontanément.

La plage des valeurs que peut prendre l'intervalle d'échappement IE est limitée d'une part par la fréquence maximale de stimulation programmée par le médecin, et d'autre part par la fréquence de base, et/ou par la valeur de la pause ventriculaire maximale autorisée, et/ou tout autre critère approprié préalablement défini.

Par ailleurs, certains événements R produits par exemple par des extrasystoles ventriculaires (ESV) peuvent conduire à un signal PEA1 de faible amplitude qui pourrait être détecté. Pour éviter les risques de stimulation dans la période vulnérable suivant un tel événement, l'intervalle d'échappement IE peut être limité à une valeur minimale, par exemple 1000 ms. Si la stimulation auriculaire antitachycardique se termine avant la fin de l'intervalle d'échappement IE_{PEA} (test 16), alors (étape 18) une stimulation auriculaire est appliquée, avec un délai (IEp_{EA} - DAV), si elle intervient à plus de 500 ms de la dernière stimulation auriculaire ; dans le cas contraire c'est une stimulation ventriculaire qui est appliquée, ceci de manière à permettre une resynchronisation satisfaisante de l'oreillette et du ventricule.

L'intervalle d'échappement est alors à nouveau calculé et géré de la manière habituelle (IE = IE_{RR}) après la fin de la stimulation auriculaire antitachycardique.

Cette situation est celle correspondant à la période comprise entre les instants t₄ et t₅ de l'exemple de la Figure 3 : le dispositif a mis fin à la stimulation auriculaire, mais il convient de resynchroniser l'oreillette et le ventricule (dans l'exemple illustré, le dispositif détecte à l'instant t₅ une dépolarisation spontanée R, révélée également par le marqueur M₅ à partir du signal EA) et de poursuivre la surveillance de l'activité ventriculaire.

## Revendications

1. Un dispositif médical actif implantable du type prothèse cardiaque de stimulation, resynchronisation et/ou défibrillation, comportant :
- des moyens de détection auriculaire, aptes à détecter la survenue d'événements auriculaires spontanés (P) ;
- des moyens de détection ventriculaire, aptes à détecter la survenue d'événements ventriculaires spontanés (R) ;
- des moyens de stimulation antitachycardique, aptes à délivrer des séquences d'impulsions de stimulation de faible énergie à une fréquence supérieure au rythme sinusal du patient en cas d'arythmie ;
- des moyens de calcul d'un intervalle d'échappement ventriculaire (IE) ;
- des moyens de stimulation ventriculaire antibradycardique, aptes à délivrer conditionnellement une impulsion de stimulation ventriculaire (V), en l'absence d'événement ventriculaire spontané (R) détecté par les moyens de détection ventriculaire à l'issue de l'intervalle d'échappement ventriculaire (IE) ;
- un capteur d'activité mécanique du myocarde, apte à délivrer un signal d'activité mécanique (EA) représentatif des mouvements produits par les contractions cycliques de la cavité ventriculaire ; et
- des moyens de détection ventriculaire comprenant :
· des moyens principaux de détection, aptes à détecter un potentiel électrique de dépolarisation ventriculaire spontanée (R), et
· des moyens auxiliaires de détection, aptes à reconnaître et isoler dans le signal d'activité mécanique délivré par le capteur une composante associée à une contraction ventriculaire,
dispositif **caractérisé en ce que** :
- les moyens de stimulation antitachycardique sont des moyens de stimulation auriculaire ;
- les moyens auxiliaires de détection sont mis en oeuvre en cas d'activation des moyens de stimulation auriculaire antitachycardique, de manière à relayer les moyens principaux de détection en présence de périodes réfractaires post-stimulation auriculaire occultant la détection de l'activité électrique du ventricule par les moyens principaux de détection ; et
- les moyens de calcul de l'intervalle d'échappement comprennent :
· des moyens pour calculer une première valeur d'intervalle d'échappement (IE_{RR}) à partir des instants successifs de survenue des potentiels de dépolarisation ventriculaire spontanée (R) détectés par les moyens principaux de détection ;
· des moyens pour calculer une seconde valeur d'intervalle d'échappement (IE_{PEA}) à partir des instants successifs de survenue desdites contractions ventriculaires détectées par les moyens auxiliaires de détection ; et
· des moyens pour sélectionner la seconde valeur d'intervalle d'échappement en cas d'activation des moyens de stimulation auriculaire antitachycardique.

2. Le dispositif de la revendication 1, dans lequel les moyens pour calculer la seconde valeur d'intervalle d'échappement sont en outre aptes à limiter la valeur calculée à une valeur minimale prédéterminée.

3. Le dispositif de la revendication 1, dans lequel les moyens pour calculer la seconde valeur d'intervalle d'échappement sont en outre aptes à moyenner une pluralité de valeurs d'échappement relevées au cours de cycles successifs.

4. Le dispositif de la revendication 1, dans lequel :
- le capteur d'activité mécanique du myocarde est un capteur d'accélération, apte à délivrer un signal d'accélération endocardiaque (EA), et
- les moyens auxiliaires de détection sont aptes à reconnaître et isoler dans le signal d'accélération endocardiaque une composante correspondant à un pic d'accélération endocardiaque (PEA1) associé à ladite contraction ventriculaire.

5. Le dispositif de la revendication 4, dans lequel le capteur d'accélération est un capteur du groupe formé par : capteur endocavitaire ; capteur épicardique ; capteur de mouvement d'une paroi du myocarde.

6. Le dispositif de la revendication 1, dans lequel le capteur d'activité mécanique du myocarde est un capteur du groupe formé par : capteur de pression ; capteur d'impédance intracardiaque.

## Claims

1. Active implantable medical device of the cardiac prosthesis type for stimulation, resynchronization and/or defibrillation, comprising:
- atrial detection means for detecting the occurrence of spontaneous atrial events (P);
- ventricular detection means for detecting the occurrence of spontaneous ventricular events (R);
- antitachycardic stimulation means for delivering sequences of low-energy stimulation pulses at a rate greater than the sinus rhythm of the patient in the case of arrhythmia;
- means for calculating a ventricular escape interval (IE);
- antibradycardic ventricular stimulation means for conditionally delivering a ventricular stimulation pulse (V), in the absence of a spontaneous ventricular event (R) detected by the ventricular detection means at the end of the ventricular escape interval (IE);
- a sensor of mechanical activity of the myocardium, for delivering a mechanical activity signal (EA) representative of the movements produced by the cyclical contractions of the ventricular cavity; and
- ventricular detection means comprising:
• main detection means for detecting an electrical potential of spontaneous ventricular depolarization (R), and
• auxiliary detection means for recognizing, and isolating from the mechanical activity signal delivered by the sensor, a component associated with a ventricular contraction,
the device being **characterized in that**:
- the antitachycardic stimulation means are atrial stimulation means;
- the auxiliary detection means are used in the case of activation of the antitachycardic atrial stimulation means, in such a way as to take over from the main detection means in the presence of post-atrial-stimulation refractory periods concealing the detection of the electrical activity of the ventricle by the main detection means; and
- the means for calculating the escape interval comprise:
• means for calculating a first escape interval value (IE_{RR}) from the successive instants of occurrence of the potentials of spontaneous ventricular depolarization (R) that are detected by the main detection means;
• means for calculating a second escape interval value (IE_{PEA}) from the successive instants of occurrence of said ventricular contractions that are detected by the auxiliary detection means; and
• means for selecting the second escape interval value in the case of activation of the antitachycardic atrial stimulation means.

2. Device according to Claim 1, in which the means for calculating the second escape interval value are additionally able to limit the calculated value to a predetermined minimum value.

3. Device according to Claim 1, in which the means for calculating the second escape interval value are additionally able to average a plurality of escape values identified during successive cycles.

4. Device according to Claim 1, in which:
- the sensor of mechanical activity of the myocardium is an acceleration sensor for delivering an endocardial acceleration signal (EA), and
- the auxiliary detection means are able to recognize, and isolate from the endocardial acceleration signal, a component corresponding to an endocardial acceleration peak (PEA1) associated with said ventricular contraction.

5. Device according to Claim 4, in which the acceleration sensor is a sensor from the group formed by: endocardial sensor; epicardial sensor; sensor of movement of a wall of the myocardium.

6. Device according to Claim 1, in which the sensor of mechanical activity of the myocardium is a sensor from the group formed by: pressure sensor; intracardiac impedance sensor.

## Patentansprüche

1. Implantierbare aktive medizinische Vorrichtung des Typs Herzprothese für eine Stimulation, Resynchronisation und/oder Defibrillation, mit:
- Mitteln zur atrialen Detektion, die das Auftreten spontaner atrialer Ereignisse (P) detektieren können;
- Mitteln zur ventrikulären Detektion, die das Auftreten spontaner ventrikulärer Ereignisse (R) detektieren können;
- Mitteln zur antitachykardialen Stimulation, die im Fall einer Arrhythmie Folgen von Stimulationsimpulsen mit geringer Energie mit einer Frequenz oberhalb des Sinusrhythmus des Patienten liefern können;
- Mitteln zum Berechnen eines ventrikulären Ausreißerintervalls (IE);
- Mitteln zur antibradykardialen ventrikulären Stimulation, die bei Ausgabe des ventrikulären Ausreißerintervalls (IE) bedingt einen Impuls (V) zur ventrikulären Stimulation bei Abwesenheit eines spontanen ventrikulären Ereignisses (R), das durch die Mittel zur ventrikulären Detektion detektiert wird, liefern können;
- einem Sensor für eine mechanische Aktivität des Myokards, der ein Signal (EA) einer mechanischen Aktivität, das Bewegungen repräsentiert, die durch zyklische Kontraktionen der ventrikulären Kammer erzeugt werden, liefern kann; und
- Mitteln zur ventrikulären Detektion, die enthalten:
• Hauptdetektionsmittel, die ein elektrisches Potential einer spontanen ventrikulären Depolarisation (R) detektieren können, und
• Hilfsdetektionsmittel, die in dem Signal einer mechanischen Aktivität, das von dem Sensor geliefert wird, eine einer ventrikulären Kontraktion zugeordnete Komponente erkennen und isolieren können,
wobei die Vorrichtung **dadurch gekennzeichnet ist, dass**:
- die Mittel zur antitachykardialen Stimulation Mittel zur atrialen Stimulation sind;
- die Hilfsdetektionsmittel im Fall einer Aktivierung der Mittel zur antitachykardialen atrialen Stimulation betätigt werden, derart, dass die Hauptdetektionsmittel in Gegenwart von refraktären Perioden einer atrialen Nachstimulation, die die Detektion einer elektrischen Aktivität des Ventrikels durch die Hauptdetektionsmittel verbergen, abgelöst werden; und
- die Mittel zum Berechnen des Ausreißerintervalls enthalten:
• Mittel, um einen ersten Wert eines Ausreißerintervalls (IE_{RR}) anhand aufeinander folgender Zeitpunkte des Auftretens von Potentialen einer spontanen ventrikulären Depolarisation (R), die durch die Hauptdetektionsmittel detektiert werden, zu berechnen;
• Mittel, um einen zweiten Wert eines Ausreißerintervalls (IE_{PEA}) anhand aufeinander folgende Zeitpunkte des Auftretens der durch die Hilfsdetektionsmittel detektierten ventrikulären Kontraktionen zu berechnen; und
• Mittel, um den zweiten Wert des Ausreißerintervalls im Fall der Aktivierung der Mittel zur antitachykardialen atrialen Stimulation auszuwählen.

2. Vorrichtung nach Anspruch 1, wobei die Mittel zum Berechnen des zweiten Werts des Ausreißerintervalls außerdem den berechneten Wert auf einen vorgegebenen Minimalwert begrenzen können.

3. Vorrichtung nach Anspruch 1, wobei die Mittel zum Berechnen des zweiten Werts des Ausreißerintervalls außerdem den Mittelwert für mehrere Ausreißerwerte, die während aufeinander folgender Zyklen abgegriffen werden, bilden können.

4. Vorrichtung nach Anspruch 1, wobei:
- der Sensor für eine mechanische Aktivität des Myokards ein Beschleunigungssensor ist, der ein endokardiales Beschleunigungssignal (EA) liefern kann, und
- die Hilfsdetektionsmittel in dem endokardialen Beschleunigungssignal eine Komponente erkennen und isolieren können, die einer Spitze (PEA1) einer endokardialen Beschleunigung entspricht, die der ventrikulären Kontraktion zugeordnet ist.

5. Vorrichtung nach Anspruch 4, wobei der Beschleunigungssensor ein Sensor aus der Gruppe ist, die gebildet ist durch: endokavitärer Sensor; epikardialer Sensor; Sensor für die Bewegung einer Myokardwand.

6. Vorrichtung nach Anspruch 1, wobei der Sensor für eine mechanische Aktivität des Myokards ein Sensor aus der Gruppe ist, die gebildet ist durch: Drucksensor; Sensor für intrakardiale Impedanz.
